# EUROPEAN PATENT APPLICATION

(11) **EP 1 084 728 A1**
(43) Date of publication of application: **21.03.2001**
(21) Application number: 00650134.0
(22) Date of filing: 15.09.2000
(51) Int. Cl.: A61M 25/10, A61M 25/00, A61F 2/06

(54) **A balloon catheter**

(30) Priority: 15.09.1999 WO PCT/IE99/00094
(71) Applicant: Angiodynamics Limited, Enniscorthy, Co Wexford (IE)
(72) Inventor: Kelly, Brian John, Clifden, County Galway (IE); Jowett, Mark, Enniscorthy, County Wexford (IE)
(74) Representative: O'Brien, John Augustine

(57) **Abstract**

A balloon catheter (1) has a relatively stiff skived hypotube (5), a relatively soft distal assembly (3) and a transition section (6) to connect the distal assembly (3) to the hypotube (5). A balloon (4) is mounted to the distal assembly (3) for use in an angioplasty procedure or during deployment of a stent. The transition (6) comprises an inner tube (9), a transition reinforcement tube (10) and an outer reinforcement tube (13). A proximal looped end (11) of a stabilising wire (12) is bonded between the inner tube (9) and the transition reinforcement tube (10) in a bonding region. The stabilising wire (12) extends from the transition section (6) to a distal end (32) located in the distal assembly (3). The catheter (1) has an inflation conduit (16) and a rapid exchange port (7) for receiving a rapid exchange guidewire. The distal assembly (3) comprises two coaxial distal tubes (14,15), which are spaced-apart to define therebetween a lumen (19) through which air for inflation of the balloon (4) is passed. The low profile transition (6) improves the kink resistance and inflation/deflation characteristics of the catheter (1).

## Description

### Introduction

The invention relates to a balloon catheter. In particular the invention relates to a rapid exchange balloon catheter.

A catheter is a tube-like member, which may be inserted into the body for a diagnostic or therapeutic reason. An example of a procedure applicable to the invention is percutaneous transluminal coronary angioplasty (PTCA), which is commonly carried out to reduce atherosclerotic plaque or cholesterol fat build up. A moveable guidewire system is used to track the catheter to the region of stenosis, after which a balloon at the distal end of the catheter is inflated. This causes the vasculature at the stenosed region to widen. The balloon is then deflated and the catheter withdrawn over the guidewire.

The invention may also be applied for deployment of a stent. A stent is typically mounted on a balloon for delivery to the region of stenosis, where the stent is deployed by inflation of the balloon.

It is known to attach a soft distal assembly section of the catheter to a stiff proximal shaft section or hypotube, so that the hypotube supports the distal assembly. A stabilising wire may be connected between the distal assembly and the hypotube. A hole is cut in the hypotube wall at its distal end and the proximal end of the stabilising wire is led out through the hole. The proximal end of the stabilising wire is attached to the hypotube by brazing, soldering or gluing it in position. This process may result in an uneven outer surface of the hypotube caused by projections of solder or glue. In addition, this process results in locally increased stiffness in the area where the stabilising wire is brazed, soldered or glued to the hypotube. Thus in general handling of the catheter, during introduction of the catheter into a guide catheter, or as the catheter passes around bends or through regions of stenosis, there is a risk of kinking. This adversely affects the catheter mobility and the inflation/deflation characteristics. Such areas of weakness also lead to a loss of torque transmission and pushability in deployment of the catheter. In addition, the inflation/deflation characteristics of such a catheter may be adversely affected because the stabilising wire partially occludes the inflation conduit.

In other cases a smaller diameter stabilising wire is used and large amounts of polymeric material are built up over the stabilising wire on the outside of the hypotube to attach the stabilising wire. This however, results in an increase in the profile of the catheter. Further, such an arrangement may result in areas of weakness in the transition between the stiff hypotube and the soft distal assembly which may cause kinking.

There is therefore a need for an improved balloon catheter, which will overcome at least some of these problems.

### Statements of Invention

According to the invention there is provided balloon catheter comprising: -
a stiff proximal shaft having a proximal end, a distal end, and a lumen extending axially therethrough, the distal end of the proximal shaft being skived axially to define a partially open distal region;
a soft distal assembly having an inflation lumen and a guidewire lumen, the inflation lumen being in fluid communication with the lumen of the proximal shaft; and
a transition section extending between the distal region of the proximal shaft and the distal assembly;
the transition section comprising: -
   an inner transition portion located within the distal region of the proximal shaft, the inner transition portion defining a transition lumen in fluid communication between the lumen of the proximal shaft and the inflation lumen of the distal assembly;
   an outer transition portion located outside the distal region of the proximal shaft; and
   a stabilising wire having a proximal end in the transition section and distal end located in the distal assembly;
   the inner and outer transition portions being fused together to form a fused kink resistant transition section.

In one embodiment of the invention the distal region of the proximal shaft extends at least partially into the distal assembly to support the distal assembly.

In a preferred embodiment the proximal end of the stabilising wire is bonded between the inner and outer transition portions. Preferably the stabilising wire extends outside the transition lumen along the length of the transition section. The stabilising wire does not occlude the transition lumen. Ideally the stabilising wire extends distally of the distal end of the proximal shaft. In this way the stabilising wire provides support for the soft distal assembly.

In another embodiment of the invention the stabilising wire is turned at least partially about a longitudinal axis of the catheter as it extends into the distal assembly. Ideally the stabilising wire is turned through an angle of approximately 90° between the proximal end and the distal end of the wire.

Desirably the proximal end of the stabilising wire is looped. In this manner the stability of the stabilising wire is enhanced.

In one embodiment of the invention the inner portion of the transition section comprises a tube located within the distal region of the proximal shaft, the tube defining the transition lumen.

Preferably the outer portion of the transition section comprises a tube extending distally from a location proximal of the distal region of the proximal shaft.

In a particularly preferred embodiment the inner and outer transition portions are fused together in separate fused regions to form the fused transition section. Ideally the separate fused regions are spaced-apart axially. This ensures the transition is a flexible fused kink resistant section.

In another preferred embodiment the outer portion of the transition section comprises two tubes, a first tube fused to the inner portion of the transition section in a first fused region, and a second tube fused to the inner portion of the transition section in a second fused region. Preferably the first and second tubes are coaxially aligned with the first tube surrounding the second tube. Ideally the first fused region is distal to the second fused region. Most preferably the transition section is bonded to the proximal shaft at each fused region. This ensures the transition section is securely attached to the proximal shaft.

In another embodiment of the invention the transition section is fused to the proximal shaft proximal of the distal region of the shaft.

In a further embodiment of the invention the transition section is fused to the distal assembly at a proximal end of the distal assembly.

In a particularly preferred embodiment the guidewire lumen is a rapid exchange guidewire lumen and the catheter includes a rapid exchange guidewire port. Preferably the distal region of the proximal shaft extends at least partially distally of the rapid exchange port to provide support in the region of the rapid exchange port. Ideally the proximal end of the stabilising wire is located in the transition section proximal of the rapid exchange port. The stabilising wire may be located in the transition section distal of the rapid exchange port.

In a further preferred embodiment the proximal shaft includes a guidewire lumen through which a guidewire may pass.

Desirably the proximal shaft is a hypotube. The proximal shaft may be of a metallic material. Alternatively the proximal shaft may be of a polymeric material. In one embodiment the proximal shaft is of a reinforced polymeric material.

Ideally the distal assembly is of a polymeric material. Desirably the transition section is of a polymeric material. Most preferably the transition section is of high density polyethylene.

Preferably the stabilising wire has a thickness of from 0.11 mm to 0.14 mm. Most preferably the stabilising wire has a thickness of approximately 0.125 mm.

In another aspect the invention provides a method for manufacturing a balloon catheter, the method comprising the steps of: -
providing a stiff proximal shaft having a proximal end and a distal end;
skiving the distal end of the proximal shaft to define a partially open distal region;
locating an inner portion of a transition section within the distal region of the proximal shaft;
locating an outer portion of the transition section outside the distal region of the proximal shaft;
locating a proximal end of a stabilising wire between the inner and outer transition portions;
fusing the inner and outer transition portions together to form a fused kink resistant transition section;
providing a soft distal assembly having a proximal end and a distal end; and
mating the distal end of the proximal shaft with the proximal end of the distal assembly.

In one embodiment of the invention the inner and outer transition portions are fused together in separate fusing regions to form the fused transition section. In this way a flexible fused kink resistant transition section is formed.

In another embodiment of the invention the method includes the steps of: -
inserting a mandrel into the inner portion of the transition section; and
removing the mandrel after fusing the inner and outer transition portions together to define a lumen through the transition section.

In a preferred embodiment the distal region of the proximal shaft is bonded within the transition section during fusion of the inner and outer transition portions. This secures the transition section to the stiff proximal shaft.

Preferably the method includes the step of bonding the outer transition portion to the proximal shaft proximal of the partially open distal region. This provides a secure attachment of the transition section to the proximal shaft.

Most preferably the distal end of the proximal shaft extends into the distal assembly during mating of the proximal shaft and the distal assembly. The stiff proximal shaft provides support for the soft distal assembly in this way.

In another preferred embodiment the stabilising wire is bonded within the transition section during fusion of the inner and outer transition portions. This secures the stabilising wire within the transition section. Ideally a distal end of the stabilising wire extends into the distal assembly during mating of the proximal shaft and the distal assembly. In this manner the stabilising wire provides support for the soft distal assembly.

In one embodiment of the invention the inner and outer transition portions are fused together by thermal bonding. Preferably the method includes the step of positioning a heat shrink tube around the transition section to assist thermal bonding of the inner and outer transition portions. Ideally the method includes the step of locating a mandrel between the heat shrink tube and the transition section to assist thermal bonding of the inner and outer transition portions.

Desirably the method includes the step of fusing the transition section to the proximal end of the distal assembly. This secures the distal assembly to the stiff proximal shaft.

In a particularly preferred embodiment the method includes the steps of: -
inserting a mandrel into an inner tube in the distal assembly before mating the proximal shaft with the distal assembly;
manipulating the mandrel; and
removing the mandrel after mating the proximal shaft with the distal assembly to define the inner tube opening as a rapid exchange guidewire port.

The distal end of the proximal shaft may be skived by a grinding process. Alternatively the distal end of the proximal shaft may be skived by a forming process. In another alternative the distal end of the proximal shaft may be skived by a moulding process. In a further alternative the distal end of the proximal shaft may be skived by a cutting process. In yet another alternative the distal end of the proximal shaft may be skived by an etching process.

### Brief Description of the Drawings

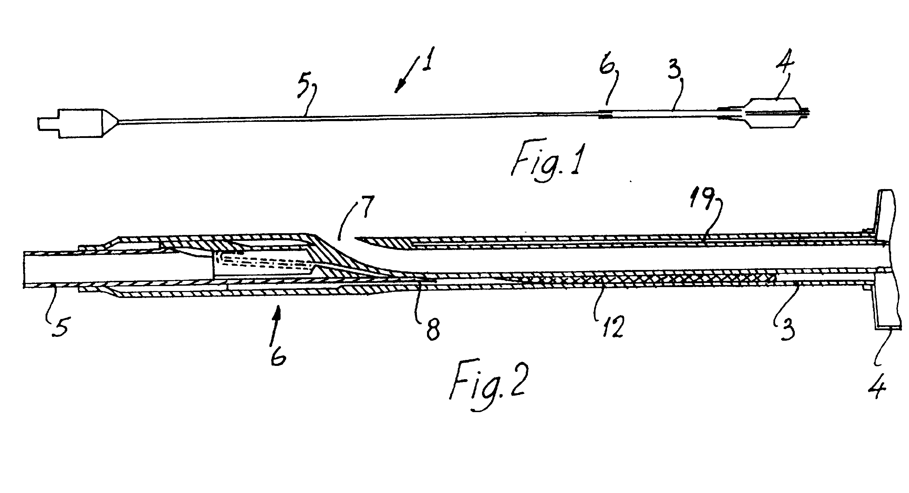

The invention will be more clearly understood from the following description thereof given by way of example only with reference to the accompanying drawings, in which:-
Fig. 1 is a schematic side view of an balloon catheter according to the invention;
Fig. 2 is an enlarged cross sectional side view of a transition section of the balloon catheter of Fig. 1;
Fig. 3 is an enlarged view of a proximal region of the transition section of Fig. 2;
Fig. 4 is a cross sectional view taken along the line F-F in Fig. 3;
Fig. 5 is a cross sectional view taken along the line E-E in Fig. 3;
Fig. 6 is a cross sectional view taken along the line D-D in Fig. 3;
Fig. 7 is an enlarged view of a central region of the transition section of Fig. 2;
Fig. 8 is a cross sectional view taken along the line E-E in Fig. 7;
Fig. 9 is a cross sectional view taken along the line D-D in Fig. 7;
Fig. 10 is a cross sectional view taken along the line C-C in Fig. 7;
Fig. 11 is a cross sectional view taken along the line B-B in Fig. 7;
Fig. 12 is an enlarged view of a distal region of the transition section of Fig. 2 ;
Fig. 13 is a cross sectional view taken along the line C-C in Fig. 12;
Fig. 14 is a cross sectional view taken along the line B-B in Fig. 12;
Fig. 15 is a cross sectional view taken along the line A-A in Fig. 12;
Figs. 16 to 18 are cross sectional side views illustrating assembly of the transition section of Fig. 2 prior to primary bonding;
Fig. 19 is a cross sectional side view of the transition section of Fig. 2 after primary bonding;
Fig. 20 is a cross sectional side view of the transition section of Fig. 2 prior to secondary bonding;
Fig. 21 is a side view of the transition section of Fig. 2 prior to secondary bonding;
Figs. 22 and 23 are side views illustrating secondary bonding of the transition section of Fig. 2;
Fig. 24 is a side view of the transition section of Fig. 2 after secondary bonding;
Fig. 25 is a cross sectional view taken along the line G-G in Fig. 24;

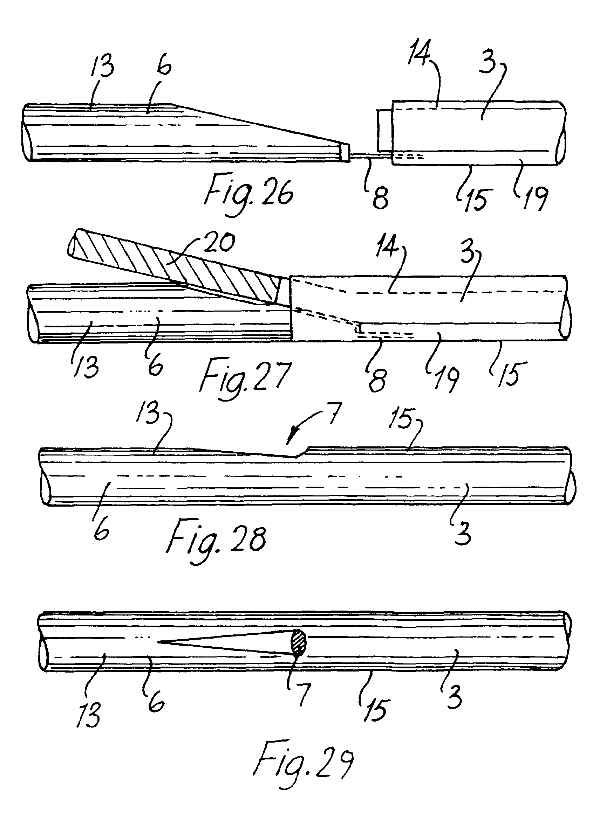

Figs. 26 and 27 are side views illustrating mating of the transition section and a distal assembly of the balloon catheter prior to tertiary bonding;
Fig. 28 is a side view of the transition section and the distal assembly after tertiary bonding; and
Fig. 29 is a plan view of the transition section and the distal assembly after tertiary bonding.

### Detailed Description

Referring to the drawings there is illustrated a balloon catheter 1 according to the invention. The balloon catheter 1 may used in treating an area of stenosis either by an angioplasty procedure or to deploy a stent.

The catheter 1 comprises a relatively stiff proximal shaft defined by a hypotube 5, a relatively soft distal assembly 3 and a transition section 6 connecting the distal assembly 3 to the hypotube 5. A balloon 4 is typically mounted to the distal assembly 3 for use in an angioplasty procedure or during deployment of a stent.

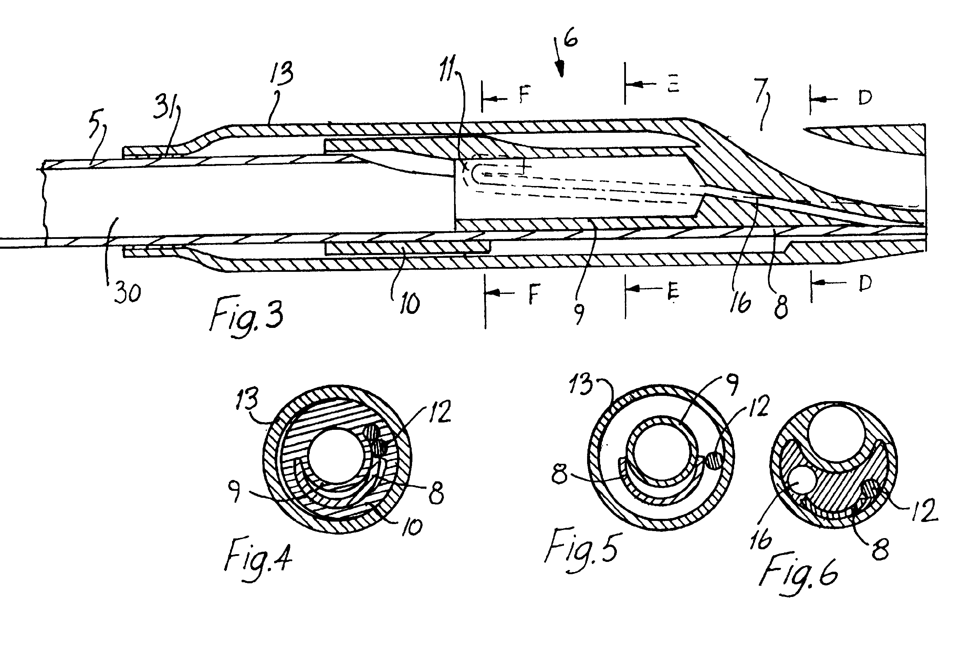

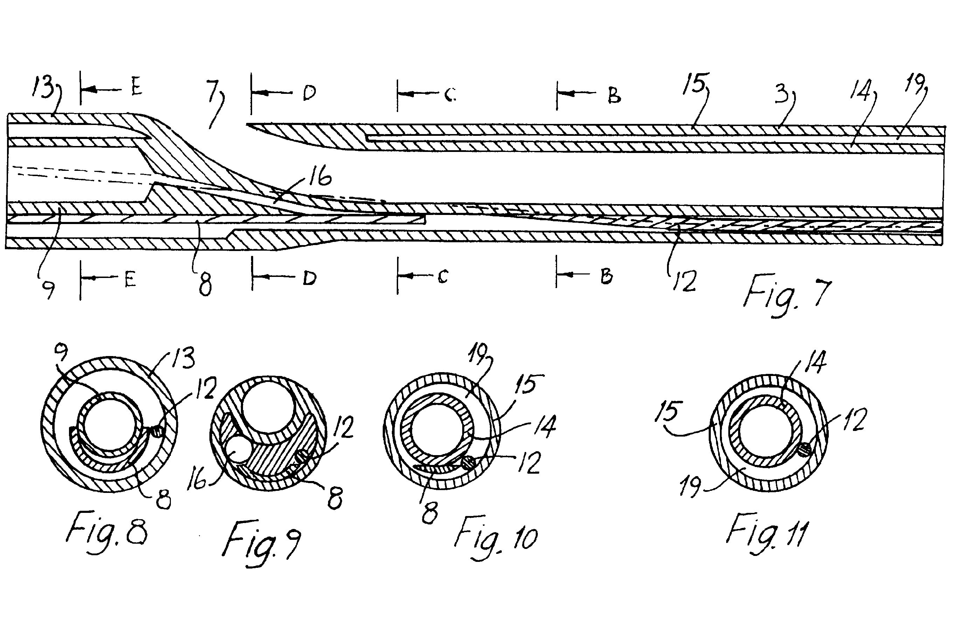

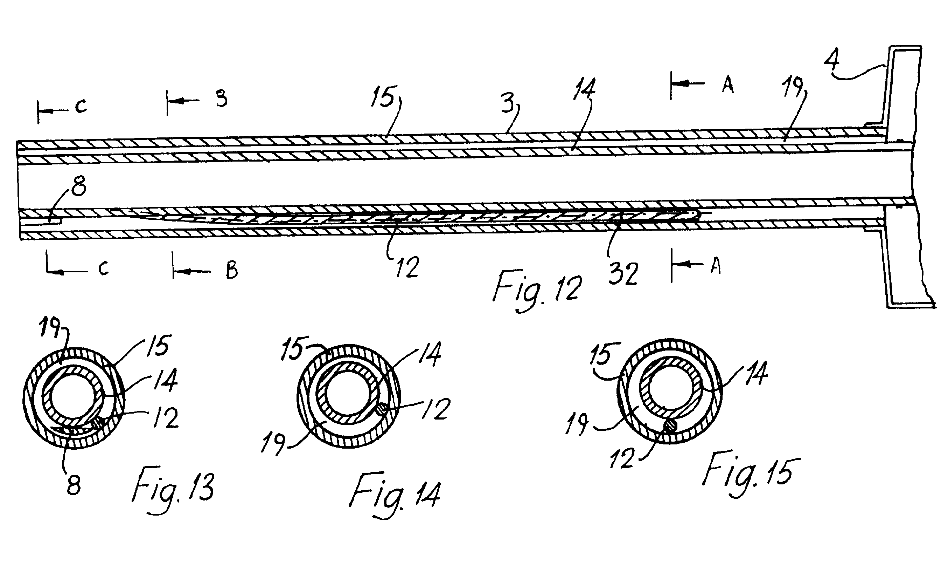

Referring in particular to Figs. 3 to 15 the transition section 6 is shown in detail. The side views in Figs. 3, 7 and 12 include gaps between the elements of the balloon catheter for ease of illustration. The cross sectional views in Figs. 4 to 6, 8 to 11 and 13 to 15 are a more accurate representation however of the relative dimensions of the catheter elements.

In this case the catheter 1 has a rapid exchange guidewire port 7 for receiving a rapid exchange guidewire (not shown).

The hypotube 5 is skived axially at its distal end for a length of from 60mm to 150mm, in this case about 100mm to define a partially open distal region or skive 8 of the hypotube. On assembly, the hypotube skive 8 extends partially into the distal assembly 3.

The term skived as used in this specification refers to a hypotube with a tapered, sliced distal end to define a partially open distal region. The term bonding as used in this specification refers in particular but not exclusively to thermal bonding. The term bond refers in particular but not exclusively to an adhesive bond. The term bond also refers to a fused or encapsulated joint.

The transition section 6 has an inner portion defined by an inner tube 9 located within the distal region 8 of the hypotube 5 and an outer transition portion including a transition reinforcement tube 10 located outside the distal region 8 of the hypotube 5. A stabilising wire 12 has a proximal end 11 in the transition section and a distal end 32 located in the distal assembly 3. The thickness of the stabilising wire is in the range of from 0.11 mm to 0.14 mm, preferably approximately 0.125 mm.

The inner tube 9 is located in the skive 8 intermediate the ends thereof. The transition reinforcement tube 10 extends from the main body of the hypotube 5, partially over the skive 8 and surrounds part of the inner tube 9. The proximal end 11 of the stabilising wire 12 is looped for added stability and is bonded between the inner tube 9 and the transition reinforcement tube 10 as illustrated in Fig. 4. The outer transition section also includes an outer reinforcement tube 13 which extends over the transition reinforcement tube 10, the inner tube 9 and a portion of the skive 8.

The distal assembly 3 comprises inner and outer coaxial distal tubes 14, 15 which are spaced-apart to define therebetween a lumen 19 through which air for inflation of the balloon 4 is passed. The inner tube 14 is bent upwardly to define the rapid exchange port 7 at a proximal end of the distal assembly 3, and the rapid exchange guidewire (not shown) is passed through the inner tube 14.

The balloon catheter has an inflation conduit 16 for inflating and deflating the balloon 4. The inflation conduit 16 is defined by a hypotube lumen 30and the inflation lumen 19 of the distal assembly which are interconnected by a transition lumen defined by the inner transition tube 9.

The stabilising wire 12 extends through the transition 6 into the lumen 19 of the distal assembly 3 in a helical arrangement. The stabilising wire 12 is turned through an angle of approximately 90° as it extends from the proximal end 11 to a distal end 32 to avoid obstructing the inflation conduit 16, and to avoid the path of the skive 8. The distal end 32 lies between but is not attached to the inner distal tube 14 or the outer distal tube 15. The stabilising wire 12 provides support for the soft distal assembly 3.

The inner tube 9, the transition reinforcement tube 10 and the outer reinforcement tube 13 are of a suitable thermoplastic material, such as high density polyethylene (HDPE).

The balloon catheter 1 is assembled in the following manner.

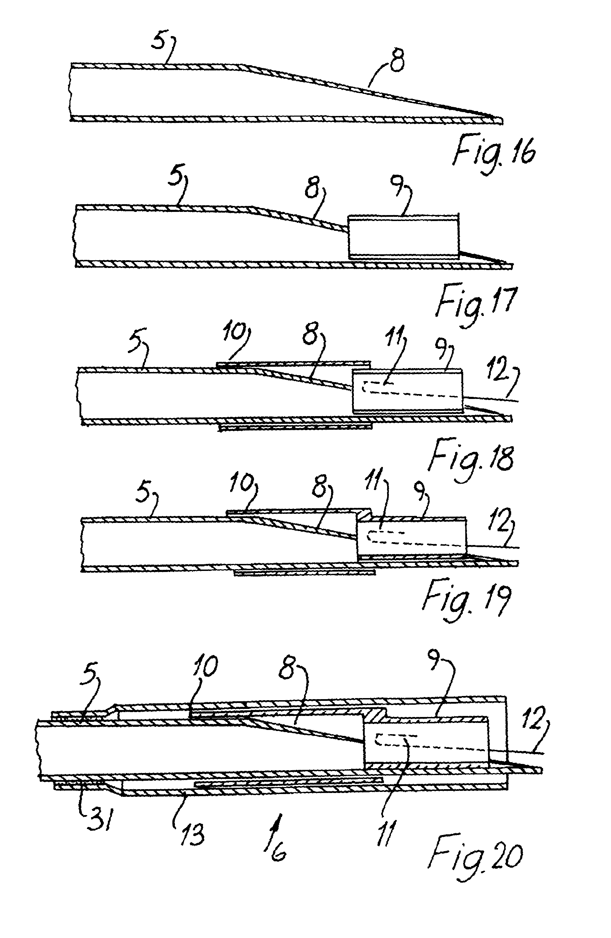

The hypotube 5 is skived at its distal end, as illustrated in Fig. 16, and the inner tube 9 is inserted into the skive 8, Fig. 17. Next the transition reinforcement tube 10 is positioned around the hypotube 5 and the inner tube 9, and the stabilising wire 12 is positioned with its proximal looped end 11 between the inner tube 9 and the transition reinforcement tube 10, Fig. 18. The inner reinforcement tube 10 extends from a point proximal of the skive 8 to a point adjacent the looped end 11.

In a primary bonding step the transition reinforcement tube 10 and the inner tube 9 are fused together into a mass of polyethylene tubing by heating the transition 6 in the region of the looped end 11, Fig. 19. The skive 8 and the stabilising wire 12 are thereby encapsulated within the fused transition.

The outer reinforcement tube 13 is then positioned around the hypotube 5, the transition reinforcement tube 10 and the inner tube 9, as illustrated in Fig. 20. The outer reinforcement tube 13 is adhesively bonded 31 to the hypotube 5 proximally. As illustrated in Fig. 4, the outer reinforcement tube 13 is not attached to the hypotube 5 or the transition reinforcement tube 10 in the region of the looped end 11 of the stabilising wire 12.

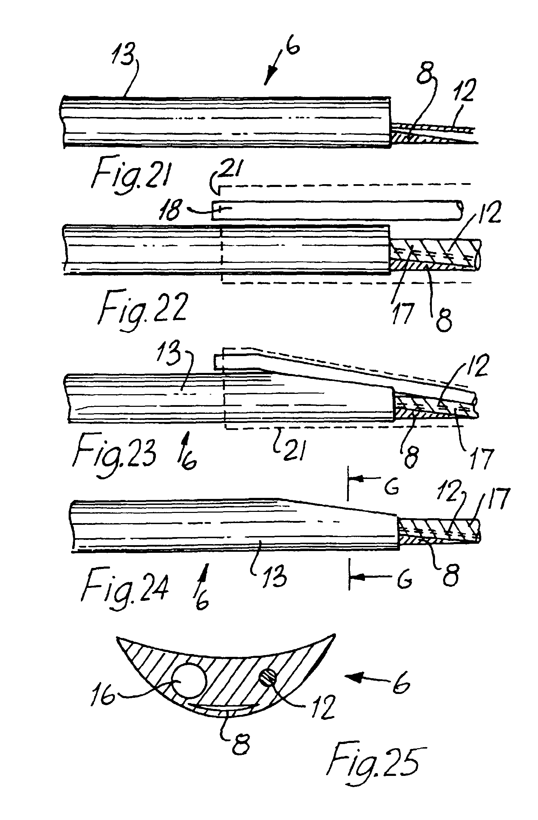

In a secondary bonding step, a first mandrel 17 is inserted into the inner tube 9 from the distal end. A heat shrink tube 21 is positioned surrounding the distal end of the transition section 6, and a second mandrel 18 is positioned within the heat shrink tube 21 parallel to the hypotube 5, along the top of the transition section 6, as illustrated in Fig. 22. The transition assembly is fused together by heating it in the region of the distal end of the transition section 6. As the transition assembly fuses the contraction of the heat shrink tube 21 pushes the second mandrel 18 down fusing the inner tube 9 and the outer reinforcement tube 13 together into a U-shaped cross section, Figs. 23 and 25. The skive 8 and the stabilising wire 12 are encapsulated within the fused transition After this secondary bonding step the heat shrink tube 21 and the second mandrel 18 are removed. The heat shrink tube 21, which is removed after the secondary bonding stage, is of a suitable thermoplastic material, such as polytetrafluoroethylene (Teflon).

The distal assembly 3 is then positioned, such that its proximal end is adjacent and in line with the distal end of the transition section 6, and the two assemblies are mated together. On assembly the hypotube skive 8, the stabilising wire 12 and the first mandrel 17 extend into the distal assembly 3. A third mandrel 20 is inserted into the proximal end opening of the inner distal tube 14, and moved, so that the inner distal tube 14 is bent upwardly to the top of the outer distal tube 15.

The mating of the assemblies is illustrated in Fig. 27. For convenience the stabilising wire 12 and the first mandrel 17 have been omitted from Fig. 27.

In a tertiary bonding step, the mated assemblies are fused together by heating in the region of the proximal end of the distal assembly 3. The mandrels 20, 17 are then removed to define a rapid exchange port 7 (which enables access to the inner distal tube 14) and an inflation conduit 16 (for inflation of the balloon 4) respectively.

The invention allows a stiff hypotube of a balloon catheter to be securely attached to a relatively soft distal assembly with a smooth and effective transition. The large bonded mass at the transition prevents any kinking of the catheter, thus facilitating torque transmission and pushability.

The hypotube skive is encapsulated between an inner portion of the transition and an outer portion of the transition in two bonding regions spaced apart axially. This enables movement of the members even when the transition is bent.

The skive of the stiff hypotube extends into the soft distal assembly to ensure a gradual change in catheter stiffness along the axial length of the catheter. The stabilising wire extends into the soft distal assembly distally of the skive to effectively form an extension of the skive. In this way discontinuities in the stiffness of the catheter are minimised to prevent kinking.

The skive of the stiff hypotube extends distally of the rapid exchange port to support the transition and the distal assembly in the region of the rapid exchange port.

The stabilising wire extends through the transition spaced apart from the inflation conduit. In this manner the inflation/deflation characteristics of the catheter are maximised because the stabilising wire does not occlude the inflation conduit.

The fused transition provides support and stability for the stabilising wire which extends into the distal assembly to support the distal assembly.

The configuration of the stabilising wire, the inner tube, the transition reinforcement tube and the outer reinforcement tube in the transition that the catheter has a smooth, low profile.

The invention is not limited to the embodiments hereinbefore described which may be varied both in construction and in detail.

## Claims

1. A balloon catheter (1) comprising: -
a stiff proximal shaft (5) having a proximal end, a distal end, and a lumen (30) extending axially therethrough, the distal end of the proximal shaft (5) being skived axially to define a partially open distal region (8);
a soft distal assembly (3) having an inflation lumen (19) and a guidewire lumen, the inflation lumen (19) being in fluid communication with the lumen (30) of the proximal shaft; and
a transition section (6) extending between the distal region (8) of the proximal shaft (5) and the distal assembly (3);
the transition section (6) comprising: -
an inner transition portion (9) located within the distal region (8) of the proximal shaft (5), the inner transition portion (9) defining a transition lumen in fluid communication between the lumen (30) of the proximal shaft (5) and the inflation lumen (19) of the distal assembly (3);
an outer transition portion (10, 13) located outside the distal region (8) of the proximal shaft (5); and
a stabilising wire (12) having a proximal end (11) in the transition section (6) and to a distal end (32) located in the distal assembly (3);
the inner and outer transition portions (9, 10, 13) being fused together to form a fused kink resistant transition section (6).

2. A catheter as claimed in claim 1 wherein the distal region (8) of the proximal shaft (5) extends at least partially into the distal assembly (3) to support the distal assembly (3).

3. A catheter as claimed in claim 1 or 2 wherein the proximal end (11) of the stabilising wire (12) is bonded between the inner and outer transition portions (9, 10), preferably the stabilising wire (12) extends outside the transition lumen along the length of the transition section (6) and distally of the distal end of the proximal shaft, preferably the stabilising wire (12) is turned at least partially about a longitudinal axis of the catheter as it extends into the distal assembly (3), preferably the stabilising wire (12) is turned through an angle of approximately 90° between the proximal end (11) and the distal end (32) of the wire (12), preferably the proximal end (11) of the stabilising wire (12) is looped.

4. A catheter as claimed in any preceding claim wherein the inner portion (9) of the transition section (6) comprises a tube (9) located within the distal region (8) of the proximal shaft (5), the tube (9) defining the transition lumen, preferably the outer portion (10, 13) of the transition section comprises a tube (10) extending distally from a location proximal of the distal region (8) of the proximal shaft (5), preferably the inner (9) and outer (10) transition portions are fused together in separate fused regions to form the fused transition section (6), preferably the separate fused regions are spaced-apart axially.

5. A catheter as claimed in claim 4 wherein the outer portion (10, 13) of the transition section comprises two tubes (10, 13), a first tube (10) fused to the inner portion (9) of the transition section (6) in a first fused region, and a second tube (13) fused to the inner portion (9) of the transition section (6) in a second fused region, preferably the first and second tubes (10, 13) are coaxially aligned with the first tube (13) surrounding the second tube (10), preferably the first fused region is distal to the second fused region, and preferably the transition section (6) is bonded to the proximal shaft (5) at each fused region.

6. A catheter as claimed in any preceding claim wherein the transition section (6) is fused to the proximal shaft (5) proximal of the distal region (8) of the shaft (5), preferably the transition section (6) is fused to the distal assembly (3) at a proximal end of the distal assembly (3).

7. A catheter as claimed in any preceding claim wherein the guidewire lumen is a rapid exchange guidewire lumen and the catheter includes a rapid exchange guidewire port (7), preferably the distal region (8) of the proximal shaft (5) extends at least partially distally of the rapid exchange port (7) to provide support in the region of the rapid exchange port (7), preferably the proximal end (11) of the stabilising wire (12) is located in the transition section (6) proximal of the rapid exchange port (7), preferably the stabilising wire (12) is located in the transition section (6) distal of the rapid exchange port (7).

8. A catheter as claimed in any of claims 1 to 6 wherein the proximal shaft includes a guidewire lumen through which a guidewire may pass, preferably the proximal shaft (5) is a hypotube (5), preferably the proximal shaft is of a metallic material, or of a polymeric material, or of a reinforced polymeric material.

9. A catheter as claimed in any preceding claim wherein the distal assembly (3) is of a polymeric material, preferably the transition section (6) is of a polymeric material, preferably of high density polyethylene.

10. A catheter as claimed in any preceding claim wherein the stabilising wire (12) has a thickness of from 0.11 mm to 0.14 mm, preferably approximately 0.125 mm.

11. A method for manufacturing a balloon catheter, the method comprising the steps of: -
providing a stiff proximal shaft (5) having a proximal end and a distal end;
skiving the distal end of the proximal shaft (5) to define a partially open distal region (8);
locating an inner portion (9) of a transition section (6) within the distal region (8) of the proximal shaft (5);
locating an outer portion (10, 13) of the transition section (6) outside the distal region (8) of the proximal shaft (5);
locating a proximal end (11) of a stabilising wire (12) between the inner and outer transition portions (9, 10, 13);
fusing the inner and outer transition portions (9, 10, 13) together to form a fused kink resistant transition section (6);
providing a soft distal assembly (3) having a proximal end and a distal end; and
mating the distal end of the proximal shaft (5) with the proximal end of the distal assembly (3).

12. A method as claimed in claim 11 wherein the inner and outer transition portions (9, 10, 13) are fused together in separate fusing regions to form the fused transition section (6), and preferably the method includes the steps of: -
inserting a mandrel (17) into the inner portion (9) of the transition section; and
removing the mandrel (17) after fusing the inner and outer transition portions (9, 10, 13) together to define a lumen through the transition section (6).

13. A method as claimed in claim 11 or 12 wherein the distal region (8) of the proximal shaft (5) is bonded within the transition section (6) during fusion of the inner and outer transition portions (9, 10, 13), preferably the distal end of the proximal shaft (5) extends into the distal assembly (3) during mating of the proximal shaft (5) and the distal assembly (3), and preferably the method includes the step of bonding the outer transition portion (10, 13) to the proximal shaft (5) proximal of the partially open distal region (8).

14. A method as claimed in any of claims 11 to 13 wherein the stabilising wire (12) is bonded within the transition section (6) during fusion of the inner and outer transition portions (9, 10, 13), preferably a distal end (32) of the stabilising wire (12) extends into the distal assembly (3) during mating of the proximal shaft (5) and the distal assembly (3).

15. A method as claimed in any of claims 11 to 14 wherein the inner and outer transition portions (9, 10, 13) are fused together by thermal bonding, and preferably the method includes the steps of positioning a heat shrink tube (21) around the transition section (6), and preferably locating a mandrel (18) between the heat shrink tube (21) and the transition section (6) to assist thermal bonding of the inner and outer transition portions (9, 10, 13).

16. A method as claimed in any of claims 11 to 15 including the step of fusing the transition section (6) to the proximal end of the distal assembly (3).

17. A method as claimed in any of claims 11 to 16 including the steps of: -
inserting a mandrel (20) into an inner tube (14) in the distal assembly (3) before mating the proximal shaft (5) with the distal assembly (3);
manipulating the mandrel (20); and
removing the mandrel (20) after mating the proximal shaft (5) with the distal assembly (3) to define the inner tube opening as a rapid exchange guidewire port (7).

18. A method as claimed in any of claims 11 to 17 wherein the distal end of the proximal shaft (5) is skived by a grinding process, a forming process, a moulding process, a cutting process or an etching process.
